(19) Europäisches Patentamt
European Patent Office
Office européen des brevets

(11) **EP 2 850 596 B1**

(12) # EUROPEAN PATENT SPECIFICATION

(45) Date of publication and mention
of the grant of the patent:
**05.12.2018 Bulletin 2018/49**

(21) Application number: **13791513.8**

(22) Date of filing: **17.05.2013**

(51) Int Cl.:
**G06T 17/00** $^{(2006.01)}$  **A61B 6/02** $^{(2006.01)}$

(86) International application number:
**PCT/CA2013/000481**

(87) International publication number:
**WO 2013/170360 (21.11.2013 Gazette 2013/47)**

(54) **METHOD AND SYSTEM FOR THE THREE-DIMENSIONAL RECONSTRUCTION OF STRUCTURES**

VERFAHREN UND ANORDNUNG ZUR RÄUMLICHEN REKONSTRUKTION VON STRUKTUREN

PROCÉDÉ ET SYSTÈME POUR LA RECONSTRUCTION TRIDIMENSIONNELLE DE STRUCTURES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.05.2012 CA 2778347
18.05.2012 US 201261688629 P**

(43) Date of publication of application:
**25.03.2015 Bulletin 2015/13**

(73) Proprietor: **Eiffel Medtech Inc.
Montréal, Québec H2J 3H9 (CA)**

(72) Inventors:
• **BENAMEUR, Said
Anjou, Québec H1J 2N8 (CA)**
• **LAVOIE, Frédéric
Ste-Martine, Québec J0S 1V0 (CA)**

(74) Representative: **Patentanwaltskanzlei
Matschnig & Forsthuber OG
Biberstraße 22
Postfach 36
1010 Wien (AT)**

(56) References cited:
**WO-A1-2009/058915**

• **KOEHLER C ET AL: "Knowledge-Assisted Reconstruction of the Human Rib Cage and Lungs", IEEE COMPUTER GRAPHICS AND APPLICATIONS, IEEE SERVICE CENTER, NEW YORK, NY, US, vol. 30, no. 1, 1 January 2010 (2010-01-01), pages 17-29, XP011298854, ISSN: 0272-1716**

• **MAX MIGNOTTE: "MDS-Based Multiresolution Nonlinear Dimensionality Reduction Model for Color Image Segmentation", IEEE TRANSACTIONS ON NEURAL NETWORKS, IEEE SERVICE CENTER, PISCATAWAY, NJ, US, vol. 22, no. 3, 1 March 2011 (2011-03-01), pages 447-460, XP011349159, ISSN: 1045-9227, DOI: 10.1109/TNN.2010.2101614**

• **BENAMEUR S ET AL: "A hierarchical statistical modeling approach for the unsupervised 3-D biplanar reconstruction of the scoliotic spine", IEEE TRANSACTIONS ON BIOMEDICAL ENGINEERING, IEEE SERVICE CENTER, PISCATAWAY, NJ, USA, vol. 52, no. 12, 1 December 2005 (2005-12-01), pages 2041-2057, XP007902896, ISSN: 0018-9294**

• **BENAMEUR ET AL.: '3D Biplanar Reconstruction of Scoliotic Vertebrae Using Statistical Models' COMPUTERIZED MEDICAL IMAGING AND GRAPHICS vol. 27, 18 September 2013, pages 321 - 337, XP055163189 Retrieved from the Internet: <URL:http://www.iro.umontreal. ca/--benameus/Publications/CMICT03. pdf> [retrieved on 2013-09-18]**

• **PUJOL ET AL.: 'Texture Segmentation by Statistic Deformable Models' INTERNATIONAL JOURNAL OF IMAGE AND GRAPHICS vol. 4, no. 3, 01 July 2004, pages 433 - 452, XP055163194 Retrieved from the Internet: <URL:http://www.cvc.uab.es/--petiallTICT DM 07 Oriol Pujol.pdf> [retrieved on 2013-09-18]**

## Description

### TECHNICAL FIELD

The present disclosure relates to a method and system for the three-dimensional reconstruction of structures. More specifically, the present disclosure relates to a method and system for the three-dimensional reconstruction of anatomical structures such as bones.

### BACKGROUND

Methods of three-dimensional (3D) reconstruction associated with Computerized Tomography (CT) scan or Magnetic Resonance Imaging (MRI) systems provide accurate 3D reconstructions of human bone structures. However, CT-scan systems submit the patient to high radiation levels. In addition, a large volume of information needs to be processed and the patient is required to lie down for an extended period of time during examination, which does not allow for the observations of deformations when the patient is standing and under the influence of gravity. Although the MRI systems do not submit the patient to high radiation levels, they possess the same last two disadvantages of CT-scan systems. Another imaging technique, completely harmless to the patient is ultrasound imaging. The latter non-invasive technique, however, is not very suitable for the analysis of 3D geometry of the bone structure because they generate too much echo and measurement noise (parasitic echoes to the geometric surface shape of dense structures).

The article of C. Koehler and T. Wischgoll "Knowledge-Assisted Reconstruction of the Human Rib Cage and Lungs", IEEE Computer Graphics and Applications, vol. 30, no. 1,2010, pp. 17-29, describes reconstruction algorithms to facilitate early detection of diseases, based on approximate volumetric reconstructions by using the inverse radon transform on X-ray image pairs.

The article of M. Mignotte, "MDS-Based Multiresolution Nonlinear Dimensionality Reduction Model for Color Image Segmentation", IEEE Trans. Neural Netw., vol. 22, no. 3, 2011, pp. 447-460, describes Multi-Dimensional Scaling (MDS) for the segmentation of 2D images.

Another 3-D reconstruction technique is presented in the article of Benameur et al., "A Hierarchical Statistical Modeling Approach for the Unsupervised 3-D Biplanar Reconstruction of Scoliotic Spine", IEEE Trans. Biomed. Eng., vol. 52, no. 12, 2005, pp. 2014-2057.

The problem of 3D multi-planar reconstruction, which consists of reconstructing a 3D anatomical structure from multiple radiographic views, belongs to the class of ill-posed problems in the sense of Hadamard. For example, the acquisition of two bi-planar radiographic images provides a set of incomplete data that does not ensure the uniqueness of the solution.

Accordingly, there is a need for to a method and system for the 3D reconstruction of structures and more specifically anatomical structures that addresses the above-described shortcomings.

### SUMMARY

The present disclosure provides a method for the 3D reconstruction of a structure according to claim 1, and methods according to claims 2 and 3.

The present disclosure also provides a system for the 3D reconstruction of a structure according to claim 4, and systems according to claims 5 to 8.

The present disclosure further provides a processor executable product stored on a data storage medium according to claim 9 and configured to cause the processor to execute the steps of the method for the 3D reconstruction of a structure according to the present disclosure.

### BRIEF DESCRIPTION OF THE FIGURES

Embodiments of the disclosure will be described by way of examples only with reference to the accompanying drawing, in which:

> **FIG. 1** is a schematic representation of a three-dimensional reconstruction system in accordance with an illustrative embodiment of the present disclosure; and

> **FIG. 2** is a flow diagram of a three-dimensional reconstruction process in accordance with an illustrative embodiment of the present disclosure.

Similar references used in different Figures denote similar components.

## DETAILED DESCRIPTION

Generally stated, the non-limitative illustrative embodiment of the present disclosure provides a method and system for the three dimensional (3D) reconstruction of structures, more specifically anatomical structures such as bones. In the present disclosure, for illustrative purposes, the example of anatomical structures will be used, more precisely bones of the human body, but it is to be understood that the system and method can be used for other types of structures.

The 3D reconstruction is achieved using calibrated bi-planar images of the anatomical structure from a two dimensional (2D) imaging system and *a priori* knowledge of the overall geometrical structure of each structure, e.g. anatomical structure or bone. This knowledge comes from a surface model learning set of considerable size, allowing modeling to take into account the anatomical variability and those related to diseases of the anatomical structures, to which is applied non-linear statistical dimension reduction, for example using the Multi-Dimensional Scaling (MDS) algorithm. The MDS algorithm allows the finding of a subspace of small dimension which preserves the metric chosen in the original space. This step allows the defining of a mesh deformation in a reduced dimensional space in which each point of the grid corresponds to a surface model of the learning set. This network in turn allows the defining of a concise 3D reconstruction parametric model in which all statistically admissible deformations from the learning set will be the values of a reduced parameter vector.

The 3D reconstruction method then consists in adjusting the projections of an anatomical structure surface model with the contours and region (homogeneous zones) of the segmented images, comprising the corresponding anatomical structure, in the bi-planar images. The 3D reconstruction problem can thus be viewed as a problem of estimation of deformation parameters of a surface model or as a problem of minimizing a cost functions of dimension *d*. This minimization can be achieved by a stochastic algorithm.

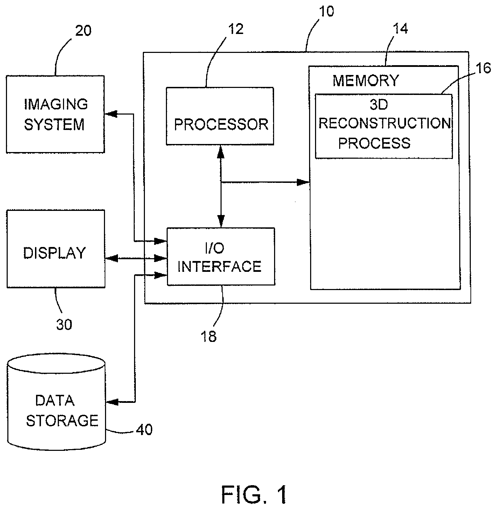

Referring to FIG. 1, the 3D reconstruction system 10 includes a processor 12 with an associated memory 14 having stored therein processor executable instructions 16 for configuring the processor 12 to perform various processes, namely the 3D reconstruction process, which process will be further described below. The image restoration system 10 further includes an input/output (I/O) interface 18 for communication with a 2D imaging system 20 and a display 30.

The 3D reconstruction system 10 obtains at least two bi-planar images of the anatomical structure from the 2D imaging system 20 and executes the 3D reconstruction process on the acquired images. The resulting 3D reconstruction is then displayed on the display 30 and may be saved to the memory 14, to other data storage devices or medium 40, or provided to a further system via the I/O interface 18.

The application of Principal Component Analysis (PCA) and/or a mixture of probabilistic PCA can be viewed as a problem of linear or locally linear dimensionality reduction. Specifically, it consists of an orthogonal projection onto a subspace of the original space.

Briefly recalling some basic ideas of the PCA, let $(x_i \in R^d)_{i=1,\cdots,l}$ be a set of observations described by d attributes. The PCA performs "optimal" graphic presentation of the observations minimizing the deformation of the cloud of points in a subspace of dimension $q < d$. Let $x$ be the matrix of observations. For centric data, the variance of the principal component $c_k = X_{p_k}$ where $p_k$ is a unit vector of the variance matrix $\Sigma = \frac{1}{l} X^t X,$ is equal to the value $\lambda_k$ associated with vector $p_k$. The main components are centered, de-correlated and are the projection of $X$ on the factorial axes of the maximum variance. PCA is a method of linear projection of the data in a space of reduced dimension. The main components are new variables formed by the linear combination of the initial variables.

Linear methods are not effective when the data are not distributed in a linear sub-space of the original space. To overcome this problem, many algorithms for non-linear dimensionality reduction have been developed. These algorithms are generally based on the idea that there is a non-linear sub-space that contains the data distribution. Among the algorithms proposed in the literature, the most popular are the Isomap, the Multi-Dimensional Scaling (MDS), Locally Linear Embedding (LLE), Semi-Definite Embedding (SDE) and t-Distributed Stochastic Neighbor Embedding (t-SNE). In the illustrative embodiment of the present disclosure, the approach used is the MDS but it is to be understood that in alternative embodiments other non-linear dimensionality reduction approaches may also be used.

In the MDS statistical approach used in the illustrative embodiment, let there be *l* observations described by *d* attributes. Let $X$ be the matrix of the observations $(x_i \in R^d)$:

$$X = \begin{pmatrix} x_{1,1} & \cdots & x_{1,d} \\ \vdots & \ddots & \vdots \\ x_{l,1} & \cdots & x_{l,d} \end{pmatrix} = \begin{pmatrix} x_1^t \\ \vdots \\ x_l^t \end{pmatrix} \qquad \textbf{Equation 1}$$

The observations can be transformed into data-centric and reduced by using the similarity between observations.

The distance matrix $\Delta$ defined by $(d_{ij})$ where $d(x_i,x_j) = ||x_i - x_j||_{l_2}$. The goal is to find a configuration of points $y_i$ with $i \in \{1,\cdots,l\}$ in a space of smaller dimension which retains the distances between initial points $x_i$. In other words, we look for $y_i \in R^q$, $q < d$ such that $d(y_i,y_j) \approx d_{ij}$ with $d_{ij} = d(x_i,x_j)$. This results in an optimization function $J$ defined by:

$$J = \sum_{i=1}^{l}\sum_{j=1}^{l} \left( d_{ij} - d(y_i - y_j) \right)^2 \qquad \textbf{Equation 2}$$

The classical MDS algorithm can be achieved either by classical optimization methods (e.g. gradient descent or equivalent) or by algebraic methods, which can be briefly recalled as:

- From the distance matrix $\Delta$, build the matrix $M$ where $M = HAH$, $H = I_l - \frac{1}{l}1_l 1_l^t$, $1_l = (1 \quad \cdots \quad 1)^t \in R^l$ et $A = -\frac{1}{2}d_{ij}^2$.

- Perform the spectral decomposition of de $M \in R^{lxl}$ where $M = W\Lambda W^t$, $W = [u_1 \cdots u_l] \in R^{lxl}$ et $\Lambda = diag(\lambda_1 \cdots \lambda_l)$. The matrix $M$ has at most \$d\$ zero eigenvalues (assuming of course that $l > d$).

- For a configuration of points in a space of reduced dimension q, simply then consider $Y = V\Lambda_d^{\frac{1}{2}}$ where $V = [u_1 \cdots u_q]$, $q < d$ is the matrix of $q$ eigenvectors and $\Lambda_q = diag(\lambda_1, \cdots \lambda_q)$ is the diagonal matrix of eigenvalues $q$. The structure of $Y$ is $Y = [y_1 \cdots y_l]^t \in R^{lxq}$ and the new variable $y^i$ is $\overline{\lambda}u_j$ where $j \in \{1, \cdots, q\}$.

The best strategies to achieve the MDS of data are to first use an algebraic method, imprecise but fast, and then use the resulting solution as initialization of an optimization method based on a gradient descent procedure or stochastic local exploration. This combination of analytical and algebraic methods of optimization provides an excellent compromise between computational speed and accuracy of the proposed solution.

The MDS is widely used for data visualization, for example, in the field of the analysis of functional Magnetic Resonance Imaging (MRI), in molecular modeling or hyper-spectral imaging. The popularity of MDS gave variants as Stochastic Proximity Embedding (SPE), Curvilinear Component Analysis (CCA), SNE and FastMap.

The 3D reconstruction process uses a segmentation method that takes into account both the notion of contour and region (detection of homogeneous zones). Cooperation between segmentation by region and contours contributes to an improved consideration of the characteristics of the anatomical structures in the images and, therefore, better segmentation thanks to the complementary nature of these two types of information. Thus, segmentation by region-contour cooperation can be expressed as a support between these two concepts in order to improve the final segmentation result.

In an anatomical structure image, only the size and more specifically the texture (i.e. distribution or statistical distribution) of the image gradient of each region present in the image is a discriminant information to distinguish bones from muscles. Indeed, the bones have a gradient distribution with uniform orientation and muscles, a distribution gradient with a longitudinal orientation (corresponding to the direction of muscle fibers) and smaller amplitude. A requantized histogram of the amplitude and direction of the gradient is a discriminant information to segment the image (i.e. differentiate the different regions), which is then used for localization and 3D reconstruction.

Accordingly, the segmentation method used by the 3D reconstruction process is based on the distribution of gradient texture and a non-linear MDS dimensionality reduction. For each pixel $(i,j)$ of each of the bi-planar images of size $LxH$ will be associated a vector of dimension $D$, having characteristics including parameters characterizing the gradient distribution of texture and orientation (considered in a small window centered about the pixel). In this context, the use of the non-linear MDS dimensionality reduction statistical method will non-linearly reduce the $LxH$ textural (gradient) vectors of dimension $D$ associated with each pixel of the images in a space of dimension $d$ (with $d \ll D$) maintaining "as best as can be" the considered distances (Euclidean or Bhattacharya). This procedure can be done using a multi-resolution approach similar to that proposed in M. Mignotte, "MDS-based multiresolution nonlinear dimensionality reduction model for color image segmentation", IEEE Transactions on Neural Networks, 22 (3) :447-460, March 2011. This dimensionality reduction will result in the optimization of a cost function where will be introduced, in addition, to reflect the contours, a weighting factor to encourage the pairing of pixels which are not separated by a contour and thus promote consistency between pixels in a region. This dimensionality reduction with, for example, $d = 1$ will lead to a viewable grayscale image where different regions appear with reduced values and very different levels of gray for which a simple K-means clustering will reliably segment (and quickly) the image into two classes.

This non-linear dimensionality step allows the defining of a deformation mesh in a reduced dimensional space in which each point of the grid will actually represent a surface model of the learning set. This in turn allows the defining of a concise 3D reconstruction parametric model in which all eligible and statistically learned deformations from the learning set will be the values of a reduced vector of d parameters.

From this deformation non-linear mesh summarizing all statistically eligible surface structures, the 3D reconstruction process consist then in the adjustment of the projections of an anatomical structure surface model (contained in this deformation mesh and by interpolation) with the contours of the segmented image, comprising the corresponding anatomical structure, by the method proposed in F. Destrempes, M. Mignotte, J.-F. Angers, "Localization of shapes using statistical models and stochastic optimization", IEEE Transactions on Pattern Analysis and Machine Intelligence, 29 (9):1603-1615, September 2007.

This 3D reconstruction process is based on a likelihood using contour pre-detection as well on a global constraint using the concept of specificity. This property is based on the fact that labels (of classes), provided by a textural pre-segmentation of the image, inside and outside of the form are different (or specific to a neighborhood of the object). This feature allows the basing of the likelihood of the Bayesian model on attributes that have the property to be very robust to noise.

The problem of 3D reconstruction is then seen as a simple problem of deformation parameters estimation of the surface model or, equivalently, as a problem of minimizing a cost function of dimension d. This minimization can be achieved by a stochastic algorithm.

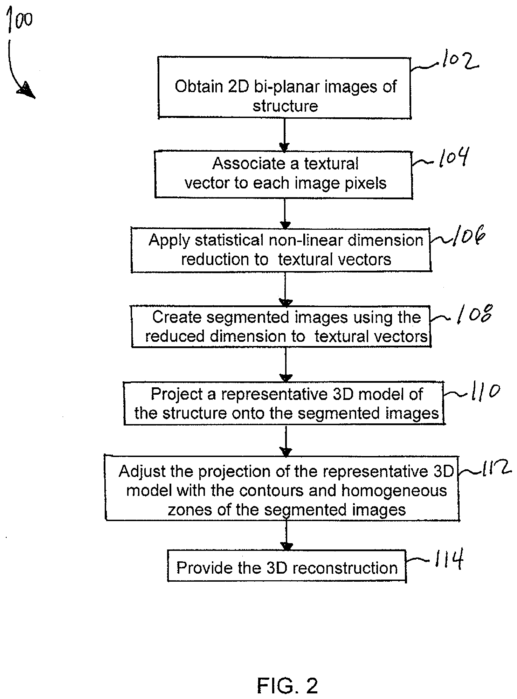

Referring to FIG. 2, there is shown a flow diagram of an illustrative example of the 3D reconstruction process 100 executed by the processor 12 (see FIG. 1). Steps of the process 100 are indicated by blocks 102 to 114.

The process 100 starts at block 102 where at least two 2D bi-planar images of a structure, for example an anatomical structure of a patient, are obtained from the imaging system 20.

Then, at block 104, a textural vector is assigned to each pixel of each of the at least two bi-planar images. The dimension D of the textural vectors on the quantization used for the gradient and orientation parameters. For example, using four (4) quantization intervals for the gradient (i.e. [0, 63], ]63, 128], ]128, 184], ]184, 255]) and four (4) quantization intervals for the orientation (i.e. [0, $\pi/4$],] $\pi/4$, $\pi/2$],] $\pi/2$, $3\pi/2$], ]$3\pi/2$, $\pi$]), each vector with be of a dimension D=16 (each combination of gradient and orientation will be assigned a value representing the number of neighboring pixels having that specific combination).

At block 106, a non-linear statistical dimensionality reduction procedure is applied to the textural vectors to reduce their dimension to $d$ for example $d$ = 2. Examples of procedures that may be used include Isomap, Multi-Dimensional Scaling, Locally Linear Embedding, Semi-Definite Embedding and t-Distributed Stochastic Neighbor Embedding.

At block 108, segmented images of the at least two calibrated 2D bi-planar images are created using the reduced dimension textural vectors.

Then, at block 110, the representative 3D model of the structure is projected onto the segmented images and, at block 112, the projection of the representative 3D model is adjusted with the contours and homogeneous zones of the segmented images using the optimization of a cost function to obtain the 3D reconstruction of the structure.

Finally, at block 114, the 3D reconstruction of the structure is provided.

The representative 3D model of the structure can be obtained using a learning set. The learning set contains, for each type of structure, a plurality of associated structure surface models. Associated with each structure surface model is a shape vector characterizing the shape of the structure surface model in 3D. A non-linear statistical dimensionality reduction procedure is then applied to the shape vectors to obtain reduced dimension shape vectors. A point in the reduced dimension space is then selected and then its corresponding reduced dimension shape vector is varied by a predefined interval. A predefined number of closest neighboring points to the selected point are then selected and the representative 3D model is then provided by interpolation using the shape vectors associated with the closest neighboring points to the selected point. It is to be understood that the predefined number of neighboring points to the selected point is a function of the degree of the interpolation.

## Claims

1. A method (100) for the 3D reconstruction of an anatomical structure, the method comprising:

   - obtaining (102) at least two calibrated 2D bi-planar images of the structure;
   - associating (104) with each pixel of each of the at least two calibrated 2D bi-planar images a textural vector characterizing the distribution of the gradient texture and orientation in a window centered around the pixel;
   - applying (106) a non-linear statistical dimensionality reduction procedure to the textural vectors to obtain reduced dimension textural vectors;
   - creating (108) segmented images of the at least two calibrated 2D bi-planar images using the reduced dimension textural vectors;
   - selecting a representative 3D model of the anatomical structure from a learning set containing a plurality of structure surface models, wherein the selection of the representative 3D model of the structure is performed by:

- associating with each structure surface model a shape vector characterizing the shape of the structure surface model in 3D;
- applying a non-linear statistical dimensionality reduction procedure to the shape vectors to obtain reduced dimension shape vectors;
- selecting a point in the reduced dimension space;
- varying the reduced dimension shape vector corresponding to the selected point by a predefined interval;
- selecting a predefined number of closest neighboring points to the selected point; and
- providing the representative 3D model by interpolation using the shape vectors associated with the closest neighboring points to the selected point;

the predefined number of closest neighboring points being a function of the degree of the interpolation.
- projecting (110) the representative 3D model onto the segmented images; and
- adjusting (112) the projection with the contours and homogeneous zones of the segmented images by minimizing a cost function of the deformation parameters of the representative 3D model to obtain the 3D reconstruction of the anatomical structure.

2. The method of claim 1, wherein the representative 3D model is selected from a plurality of proposed representative 3D models associated with respective points in the reduced dimension space.

3. The method of claim 1 or 2, wherein the non-linear statistical dimensionality reduction procedure is selected from a group consisting of Isomap, Multi-Dimensional Scaling, Locally Linear Embedding, Semi-Definite Embedding and t-Distributed Stochastic Neighbor Embedding.

4. A system (10) for the 3D reconstruction of an anatomical structure, comprising:

an input/output interface (18) configured to receive at least two calibrated 2D bi-planar images;
a database (14, 40) having stored therein a learning set containing a plurality of structure surface models; and
a processor (12) in communication with the input/output interface (18) and the database (14, 40), wherein the processor is configured for:

- associating with each pixel of each of the at least two calibrated 2D bi-planar images a textural vector characterizing the distribution of the gradient texture and orientation in a window centered around the pixel;
- applying a non-linear statistical dimensionality reduction procedure to the textural vectors to obtain reduced dimension textural vectors;
- creating segmented images of the at least two calibrated 2D bi-planar images using the reduced dimension textural vectors;
- selecting a representative 3D model of the anatomical structure from the learning set, wherein the selection of the representative 3D model of the structure is performed by:

- associating with each structure surface model a shape vector characterizing the shape of the structure surface model in 3D;
- applying a non-linear statistical dimensionality reduction procedure to the shape vectors to obtain reduced dimension shape vectors;
- selecting a point in the reduced dimension space;
- varying the reduced dimension shape vector corresponding to the selected point by a predefined interval;
- selecting a predefined number of closest neighboring points to the selected point; and
- providing the representative 3D model by interpolation using the shape vectors associated with the closest neighboring points to the selected point;

the predefined number of closest neighboring points being a function of the degree of the interpolation.
- projecting the representative 3D model onto the segmented images; and
- adjusting the projection with the contours and homogeneous zones of the segmented images by minimizing a cost function of the deformation parameters of the representative 3D model to obtain the 3D reconstruction of the anatomical structure.

**5.** The system of claim 4, further comprising a display (30) in communication with the input/output interface (18), the input/output interface (18) being further configured to provide the 3D reconstruction of the structure to the display (30).

**6.** The system of claims 4 or 5, further comprising a 2D imaging system (20) in communication with the input/output interface (18), the 2D imaging system (20) providing the at least two calibrated 2D bi-planar images to the input/output interface (18).

**7.** The system of any of claims 4 to 6, wherein the non-linear statistical dimensionality reduction procedure is selected from a group consisting of Isomap, Multi-Dimensional Scaling, Locally Linear Embedding, Semi-Definite Embedding and t-Distributed Stochastic Neighbor Embedding.

**8.** The system of any of claims 4 to 7, wherein the representative 3D model is selected from a plurality of proposed representative 3D models associated with respective points in the reduced dimension space.

**9.** A processor executable product stored on a data storage medium and configured to cause the processor to perform operations comprising:

- obtaining at least two calibrated 2D bi-planar images of an anatomical structure;
- associating with each pixel of each of the at least two calibrated 2D bi-planar images a textural vector characterizing the distribution of the gradient texture and orientation in a window centered around the pixel;
- applying a non-linear statistical dimensionality reduction procedure to the textural vectors to obtain reduced dimension textural vectors;
- creating segmented images of the at least two calibrated 2D bi-planar images using the reduced dimension textural vectors;
- selecting a representative 3D model of the anatomical structure from a learning set containing a plurality of structure surface models, wherein the selection of the representative 3D model of the structure is performed by:

- associating with each structure surface model a shape vector characterizing the shape of the structure surface model in 3D;
- applying a non-linear statistical dimensionality reduction procedure to the shape vectors to obtain reduced dimension shape vectors;
- selecting a point in the reduced dimension space;
- varying the reduced dimension shape vector corresponding to the selected point by a predefined interval;
- selecting a predefined number of closest neighboring points to the selected point; and
- providing the representative 3D model by interpolation using the shape vectors associated with the closest neighboring points to the selected point;

the predefined number of closest neighboring points being a function of the degree of the interpolation.
- projecting the representative 3D model onto the segmented images; and
- adjusting the projection with the contours and homogeneous zones of the segmented images by minimizing a cost function of the deformation parameters of the representative 3D model to obtain the 3D reconstruction of the anatomical structure.

**Patentansprüche**

**1.** Verfahren (100) zur dreidimensionalen Rekonstruktion einer anatomischen Struktur, wobei das Verfahren umfasst:

- Beschaffen (102) von zumindest zwei kalibrierten zweidimensionalen biplanaren Bildern der Struktur;
- Zuordnen (104), zu jedem Pixel aus jedem der zumindest zwei kalibrierten zweidimensionalen biplanaren Bildern, eines Texturvektors, der die Verteilung der Gradienttextur und Orientierung in einem um das Pixel zentrierten Fenster charakterisiert;
- Anwenden (106) eines Verfahrens zur nichtlinearen statistischen Dimensionalitätsreduktion auf die Texturvektoren, um Texturvektoren verringerter Dimension zu erhalten;
- Erzeugen (108) segmentierter Bilder der zumindest zwei kalibrierten zweidimensionalen biplanaren Bilder unter Verwendung der Texturvektoren verringerter Dimension;
- Auswählen eines repräsentativen dreidimensionalen Modells der anatomischen Struktur aus einer Lernmenge, die eine Vielzahl von Strukturoberflächenmodellen enthält, wobei die Auswahl des repräsentativen dreidimensionalen Modells durch

- Zuordnen eines Gestaltvektors, der die Form des Strukturoberflächenmodells in drei Dimensionen charakterisiert, zu jedem Strukturoberflächenmodell,
- Anwenden eines Verfahrens zur nichtlinearen statistischen Dimensionalitätsreduktion auf die Gestaltvektoren, um Gestaltvektoren verringerter Dimension zu erhalten,
- Auswählen eines Punkts in dem Raum verringerter Dimension,
- Abändern des Gestaltvektors verringerter Dimension, der dem ausgewählten Punkt entspricht, um ein vordefiniertes Intervall,
- Auswählen einer vorbestimmten Anzahl von Punkten, die nächste Nachbarn zum ausgewählten Punkt sind, und
- Erstellen des repräsentativen dreidimensionalen Modells durch Interpolation mithilfe der Gestaltvektoren, die den nächste-Nachbarn-Punkten zum ausgewählten Punkt zugeordnet sind,

durchgeführt wird, wobei die vorbestimmte Anzahl der nächste-Nachbarn-Punkte eine Funktion des Grads der Interpolation ist;
- Projizieren (110) des repräsentativen dreidimensionalen Modells auf die segmentierten Bilder; und
- Einstellen (112) der Projektion auf die Konturen und homogenen Bereiche der segmentierten Bilder durch Minimieren einer Kostenfunktion der Deformationparameter des repräsentativen dreidimensionalen Modells, um die dreidimensionale Rekonstruktion der anatomischen Struktur zu gewinnen.

2. Verfahren nach Anspruch 1, wobei das repräsentative dreidimensionale Modell aus einer Vielzahl vorgeschlagener repräsentativen dreidimensionalen Modelle ausgewählt wird, die jeweils Punkten in dem Raum verringerter Dimension zugeordnet sind.

3. Verfahren nach Anspruch 1 oder 2, wobei das Verfahren zur nichtlinearen statistischen Dimensionalitätsreduktion ausgewählt ist aus einer Gruppe bestehend aus Isomap, multidimensionalem Skalieren, lokaler linearer Einbettung, semidefinierter Einbettung und t-verteilter stochastischen Nachbareinbettung.

4. System (10) zur dreidimensionalen Rekonstruktion einer anatomischen Struktur, aufweisend:

eine Ein/Ausgabeschnittstelle (18) zum Empfangen von zumindest zwei kalibrierten zweidimensionalen biplanaren Bildern;
eine Datenbank (14, 40), in der eine Lernmenge, die eine Vielzahl von Strukturoberflächenmodellen enthält, gespeichert ist; und
ein Prozessor (12), der in Kommunikation mit der Ein/Ausgabeschnittstelle (18) und der Datenbank (14, 40) steht und eingerichtet ist zum:

- Zuordnen, zu jedem Pixel aus jedem der zumindest zwei kalibrierten zweidimensionalen biplanaren Bildern, eines Texturvektors, der die Verteilung der Gradienttextur und Orientierung in einem um das Pixel zentrierten Fenster charakterisiert;
- Anwenden eines Verfahrens zur nichtlinearen statistischen Dimensionalitätsreduktion auf die Texturvektoren, um Texturvektoren verringerter Dimension zu erhalten;
- Erzeugen segmentierter Bilder der zumindest zwei kalibrierten zweidimensionalen biplanaren Bilder unter Verwendung der Texturvektoren verringerter Dimension;
- Auswählen eines repräsentativen dreidimensionalen Modells der anatomischen Struktur aus der Lernmenge, wobei die Auswahl des repräsentativen dreidimensionalen Modells durch

- Zuordnen eines Gestaltvektors, der die Form des Strukturoberflächenmodells in drei Dimensionen charakterisiert, zu jedem Strukturoberflächenmodell,
- Anwenden eines Verfahrens zur nichtlinearen statistischen Dimensionalitätsreduktion auf die Gestaltvektoren, um Gestaltvektoren verringerter Dimension zu erhalten,
- Auswählen eines Punkts in dem Raum verringerter Dimension,
- Abändern des Gestaltvektors verringerter Dimension, der dem ausgewählten Punkt entspricht, um ein vordefiniertes Intervall,
- Auswählen einer vorbestimmten Anzahl von Punkten, die nächste Nachbarn zum ausgewählten Punkt sind, und
- Erstellen des repräsentativen dreidimensionalen Modells durch Interpolation mithilfe der Gestaltvektoren, die den nächste-Nachbarn-Punkten zum ausgewählten Punkt zugeordnet sind,

durchgeführt wird, wobei die vorbestimmte Anzahl der nächste-Nachbarn-Punkte eine Funktion des Grads der Interpolation ist;
- Projizieren des repräsentativen dreidimensionalen Modells auf die segmentierten Bilder; und
- Einstellen der Projektion auf die Konturen und homogenen Bereiche der segmentierten Bilder durch Minimieren einer Kostenfunktion der Deformationparameter des repräsentativen dreidimensionalen Modells, um die dreidimensionale Rekonstruktion der anatomischen Struktur zu gewinnen.

5. System nach Anspruch 4, ferner aufweisend eine Anzeige (30), die in Kommunikation mit der Ein/Ausgabeschnittstelle (18) steht, wobei die Ein/Ausgabeschnittstelle (18) ferner dazu eingerichtet ist, die dreidimensionale Rekonstruktion der Struktur an die Anzeige zu liefern.

6. System nach Anspruch 4 oder 5, ferner aufweisend ein in Kommunikation mit der Ein/Ausgabeschnittstelle (18) stehendes System zur zweidimensionalen Bildgebung, welches die zumindest zwei kalibrierten zweidimensionalen biplanaren Bilder an die Ein/Ausgabeschnittstelle (18) liefert.

7. System nach einem der Ansprüche 4 bis 6, wobei das Verfahren zur nichtlinearen statistischen Dimensionalitätsreduktion ausgewählt ist aus einer Gruppe bestehend aus Isomap, multidimensionalem Skalieren, lokaler linearer Einbettung, semidefinierter Einbettung, und t-verteilter stochastischen Nachbareinbettung.

8. System nach einem der Ansprüche 4 bis 7, wobei das repräsentative dreidimensionale Modell aus einer Vielzahl vorgeschlagener repräsentativen dreidimensionalen Modelle ausgewählt wird, die jeweils Punkten in dem Raum verringerter Dimension zugeordnet sind.

9. Prozessor-ausführbares Produkt, das auf einem Datenspeichermedium gespeichert ist und dazu eingerichtet ist, den Prozessor Arbeitsschritte ausführen zu lassen, welche umfassen:

- Beschaffen von zumindest zwei kalibrierten zweidimensionalen biplanaren Bildern einer anatomischen Struktur;
- Zuordnen, zu jedem Pixel aus jedem der zumindest zwei kalibrierten zweidimensionalen biplanaren Bildern, eines Texturvektors, der die Verteilung der Gradienttextur und Orientierung in einem um das Pixel zentrierten Fenster charakterisiert;
- Anwenden eines Verfahrens zur nichtlinearen statistischen Dimensionalitätsreduktion auf die Texturvektoren, um Texturvektoren verringerter Dimension zu erhalten;
- Erzeugen segmentierter Bilder der zumindest zwei kalibrierten zweidimensionalen biplanaren Bilder unter Verwendung der Texturvektoren verringerter Dimension;
- Auswählen eines repräsentativen dreidimensionalen Modells der anatomischen Struktur aus einer Lernmenge, die eine Vielzahl von Strukturoberflächenmodellen enthält, wobei die Auswahl des repräsentativen dreidimensionalen Modells durch

- Zuordnen eines Gestaltvektors, der die Form des Strukturoberflächenmodells in drei Dimensionen charakterisiert, zu jedem Strukturoberflächenmodell,
- Anwenden eines Verfahrens zur nichtlinearen statistischen Dimensionalitätsreduktion auf die Gestaltvektoren, um Gestaltvektoren verringerter Dimension zu erhalten,
- Auswählen eines Punkts in dem Raum verringerter Dimension,
- Abändern des Gestaltvektors verringerter Dimension, der dem ausgewählten Punkt entspricht, um ein vordefiniertes Intervall,
- Auswählen einer vorbestimmten Anzahl von Punkten, die nächste Nachbarn zum ausgewählten Punkt sind, und
- Erstellen des repräsentativen dreidimensionalen Modells durch Interpolation mithilfe der Gestaltvektoren, die den nächste-Nachbarn-Punkten zum ausgewählten Punkt zugeordnet sind,

durchgeführt wird, wobei die vorbestimmte Anzahl der nächste-Nachbarn-Punkte eine Funktion des Grads der Interpolation ist;
- Projizieren des repräsentativen dreidimensionalen Modells auf die segmentierten Bilder; und
- Einstellen der Projektion auf die Konturen und homogenen Bereiche der segmentierten Bilder durch Minimieren einer Kostenfunktion der Deformationparameter des repräsentativen dreidimensionalen Modells, um die dreidimensionale Rekonstruktion der anatomischen Struktur zu gewinnen.

**Revendications**

1. Procédé (100) pour la reconstruction 3D d'une structure anatomique, le procédé comprenant :

   - obtenir (102) au moins deux images biplanaires 2D étalonnées de la structure ;
   - associer (104) à chaque pixel de chacune des au moins deux images biplanaires 2D étalonnées un vecteur textural caractérisant la distribution de la texture de gradient et l'orientation dans une fenêtre centrée autour du pixel ;
   - appliquer (106) une procédure de réduction de dimensionnalité statistique non linéaire aux vecteurs texturaux pour obtenir des vecteurs texturaux de dimension réduite ;
   - créer (108) des images segmentées des au moins deux images biplanaires 2D étalonnées à l'aide des vecteurs texturaux de dimension réduite ;
   - sélectionner un modèle 3D représentatif de la structure anatomique à partir d'un ensemble d'apprentissage contenant une pluralité de modèles de surface de structure, la sélection du modèle 3D représentatif de la structure étant effectuée par :

      - association à chaque modèle de surface de structure d'un vecteur de forme caractérisant la forme du modèle de surface de structure en 3D ;
      - application d'une procédure de réduction de dimensionnalité statistique non linéaire aux vecteurs de forme pour obtenir des vecteurs de forme de dimension réduite ;
      - sélection d'un point dans l'espace de dimension réduite ;
      - variation d'un intervalle prédéfini du vecteur de forme de dimension réduite correspondant au point sélectionné ;
      - sélection d'un nombre prédéfini de points voisins les plus proches du point sélectionné ; et
      - fourniture du modèle 3D représentatif par interpolation à l'aide des vecteurs de forme associés aux points voisins les plus proches du point sélectionné ;

   le nombre prédéfini de points voisins les plus proches étant une fonction du degré de l'interpolation,
   - projeter (110) le modèle 3D représentatif sur les images segmentées ; et
   - ajuster (112) la projection avec les contours et les zones homogènes des images segmentées en rendant minimale une fonction de coût des paramètres de déformation du modèle 3D représentatif pour obtenir la reconstruction 3D de la structure anatomique.

2. Procédé selon la revendication 1, dans lequel le modèle 3D représentatif est sélectionné parmi une pluralité de modèles 3D représentatifs proposés associés à des points respectifs dans l'espace de dimension réduite.

3. Procédé selon l'une des revendications 1 ou 2, dans lequel la procédure de réduction de dimensionnalité statistique non linéaire est sélectionnée dans un groupe comprenant un mappage isométrique (Isomap), une mise à l'échelle multidimensionnelle, une intégration localement linéaire, une intégration semi-définie et une intégration de voisin stochastique distribuée en t.

4. Système (10) pour la reconstruction 3D d'une structure anatomique, comprenant :

   une interface d'entrée/sortie (18) configurée pour recevoir au moins deux images biplanaires 2D étalonnées ;
   une base de données (14, 40) dans laquelle est stocké un ensemble d'apprentissage contenant une pluralité de modèles de surface de structure ; et
   un processeur (12) en communication avec l'interface d'entrée/sortie (18) et la base de données (14, 40), le processeur étant configuré pour :

      - associer à chaque pixel de chacune des au moins deux images biplanaires 2D étalonnées un vecteur textural caractérisant la distribution de la texture de gradient et l'orientation dans une fenêtre centrée autour du pixel ;
      - appliquer une procédure de réduction de dimensionnalité statistique non linéaire aux vecteurs texturaux pour obtenir des vecteurs texturaux de dimension réduite ;
      - créer des images segmentées des au moins deux images biplanaires 2D étalonnées à l'aide des vecteurs texturaux de dimension réduite ;
- sélectionner un modèle 3D représentatif de la structure anatomique à partir de l'ensemble d'apprentissage, la sélection du modèle 3D représentatif de la structure étant effectuée par :

    - association à chaque modèle de surface de structure d'un vecteur de forme caractérisant la forme du modèle de surface de structure en 3D ;
    - application d'une procédure de réduction de dimensionnalité statistique non linéaire aux vecteurs de forme pour obtenir des vecteurs de forme de dimension réduite ;
    - sélection d'un point dans l'espace de dimension réduite ;
    - variation d'un intervalle prédéfini du vecteur de forme de dimension réduite correspondant au point sélectionné;
    - sélection d'un nombre prédéfini de points voisins les plus proches du point sélectionné ; et
    - fourniture du modèle 3D représentatif par interpolation à l'aide des vecteurs de forme associés aux points voisins les plus proches du point sélectionné ;

le nombre prédéfini de points voisins les plus proches étant une fonction du degré de l'interpolation,
- projeter le modèle 3D représentatif sur les images segmentées ; et
- ajuster la projection avec les contours et les zones homogènes des images segmentées en rendant minimale une fonction de coût des paramètres de déformation du modèle 3D représentatif pour obtenir la reconstruction 3D de la structure anatomique.

5. Système selon la revendication 4, comprenant en outre un dispositif d'affichage (30) en communication avec l'interface d'entrée/sortie (18), l'interface d'entrée/sortie (18) étant en outre configurée pour fournir la reconstruction 3D de la structure au dispositif d'affichage (30).

6. Système selon l'une des revendications 4 ou 5, comprenant en outre un système d'imagerie 2D (20) en communication avec l'interface d'entrée/sortie (18), le système d'imagerie 2D (20) fournissant les au moins deux images biplanaires 2D étalonnées à l'interface d'entrée/sortie (18).

7. Système selon l'une quelconque des revendications 4 à 6, dans lequel la procédure de réduction de dimensionnalité statistique non linéaire est sélectionnée dans un groupe comprenant un mappage isométrique (Isomap), une mise à l'échelle multidimensionnelle, une intégration localement linéaire, une intégration semi-définie et une intégration de voisin stochastique distribuée en t.

8. Système selon l'une quelconque des revendications 4 à 7, dans lequel le modèle 3D représentatif est sélectionné parmi une pluralité de modèles 3D représentatifs proposés associés à des points respectifs dans l'espace de dimension réduite.

9. Produit pouvant être exécuté par un processeur stocké sur un support de stockage de données et configuré pour amener le processeur à effectuer des opérations comprenant :

    - obtenir au moins deux images biplanaires 2D étalonnées d'une structure anatomique ;
    - associer à chaque pixel de chacune des au moins deux images biplanaires 2D étalonnées un vecteur textural caractérisant la distribution de la texture de gradient et l'orientation dans une fenêtre centrée autour du pixel ;
    - appliquer une procédure de réduction de dimensionnalité statistique non linéaire aux vecteurs texturaux pour obtenir des vecteurs texturaux de dimension réduite ;
    - créer des images segmentées des au moins deux images biplanaires 2D étalonnées à l'aide des vecteurs texturaux de dimension réduite ;
    - sélectionner un modèle 3D représentatif de la structure anatomique à partir d'un ensemble d'apprentissage contenant une pluralité de modèles de surface de structure, la sélection du modèle 3D représentatif de la structure étant effectuée par :

        - association à chaque modèle de surface de structure d'un vecteur de forme caractérisant la forme du modèle de surface de structure en 3D ;
        - application d'une procédure de réduction de dimensionnalité statistique non linéaire aux vecteurs de forme pour obtenir des vecteurs de forme de dimension réduite ;
        - sélection d'un point dans l'espace de dimension réduite ;
        - variation d'un intervalle prédéfini du vecteur de forme à dimension réduite correspondant au point sélectionné ;

- sélection d'un nombre prédéfini de points voisins les plus proches du point sélectionné ; et
- fourniture du modèle 3D représentatif par interpolation à l'aide des vecteurs de forme associés aux points voisins les plus proches du point sélectionné ;

le nombre prédéfini de points voisins les plus proches étant une fonction du degré de l'interpolation,
- projeter le modèle 3D représentatif sur les images segmentées ; et
- ajuster la projection avec les contours et les zones homogènes des images segmentées en rendant minimale une fonction de coût des paramètres de déformation du modèle 3D représentatif pour obtenir la reconstruction 3D de la structure anatomique.

FIG. 1

*100*

*102*

Obtain 2D bi-planar images of structure

*104*

Associate a textural vector to each image pixels

*106*

Apply statistical non-linear dimension reduction to textural vectors

*108*

Create segmented images using the reduced dimension to textural vectors

*110*

Project a representative 3D model of the structure onto the segmented images

*112*

Adjust the projection of the representative 3D model with the contours and homogeneous zones of the segmented images

*114*

Provide the 3D reconstruction

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **C. KOEHLER ; T. WISCHGOLL.** Knowledge-Assisted Reconstruction of the Human Rib Cage and Lungs. *IEEE Computer Graphics and Applications,* 2010, vol. 30 (1), 17-29
- **M. MIGNOTTE.** MDS-Based Multiresolution Nonlinear Dimensionality Reduction Model for Color Image Segmentation. *IEEE Trans. Neural Netw.,* 2011, vol. 22 (3), 447-460
- **BENAMEUR et al.** A Hierarchical Statistical Modeling Approach for the Unsupervised 3-D Biplanar Reconstruction of Scoliotic Spine. *IEEE Trans. Biomed. Eng.,* 2005, vol. 52 (12), 2014-2057

- **M. MIGNOTTE.** MDS-based multiresolution nonlinear dimensionality reduction model for color image segmentation. *IEEE Transactions on Neural Networks,* March 2011, vol. 22 (3), 447-460
- **F. DESTREMPES ; M. MIGNOTTE ; J.-F. ANGERS.** Localization of shapes using statistical models and stochastic optimization. *IEEE Transactions on Pattern Analysis and Machine Intelligence,* September 2007, vol. 29 (9), 1603-1615